# EUROPEAN PATENT SPECIFICATION

(11) **EP 3 501 507 B1**
(45) Date of publication and mention of the grant of the patent: **15.07.2020**
(21) Application number: 18213053.4
(22) Date of filing: 17.12.2018
(51) Int. Cl.: A61K 9/20, A61K 47/10, A61K 9/48, A61K 31/765, A61K 35/747, A61P 15/02, A61K 9/02

(54) **MACROGOLS FOR APPLICATION TO THE MUCOSA, AND THERAPEUTIC USES THEREOF**
MACROGOLE ZUR BEHANDLUNG DER SCHLEIMHÄUTE UND THERAPEUTISCHE VERWENDUNGEN DAVON
MACROGOLS POUR APPLICATION SUR LA MUQUEUSE ET LEURS UTILISATIONS THÉRAPEUTIQUES

(30) Priority: 21.12.2017 IT 201700148637
(43) Date of publication of application: 26.06.2019
(73) Proprietor: S.I.I.T. S.r.l.-Servizio Internazionale Imballaggi Termosaldanti, 20090 Trezzano sul Naviglio MI (IT)
(72) Inventor: MARCELLONI, Luciano, 20147 MILANO (IT); MARTINUZZI, Elena, 20090 TREZZANO SUL NAVIGLIO (MI) (IT)
(74) Representative: Bianchetti Bracco Minoja S.r.l.

(56) References cited:
- CN-A- 106 309 402
- US-A1- 2005 209 209
- US-A1- 2015 080 467
- EL-GINDY A ET AL: "MUCOADHESIVE VAGINAL TABLETS OF NATAMYCIN FOR VAGINAL CANDIDIASIS", BULLETIN OF PHARMACEUTICAL SCIEN, ASSIUT UNIVERSITY PRESS, ASSIUT, EG, vol. 26, no. PART 01, 1 June 2003 (2003-06-01), pages 29-40, XP008056653, ISSN: 1110-0052

## Description

### FIELD OF THE INVENTION

The present invention relates to pharmaceutical compositions in solid form comprising a PEG with a grade of 3000 or more, for use in the topical treatment or prevention of vaginal atrophy.

### BACKGROUND TO THE INVENTION

Women approaching the menopause are liable to suffer from dryness and hypotonia of the mucosa in the vaginal area, commonly known as vaginal atrophy, which involves unpleasant dryness and itching. Vaginal atrophy caused by a reduction in oestrogens is frequent, and involves a physiological tissue modification characterised by loss of tone and inflammation of the vaginal mucosa.

A number of effective preparations which can be used for this problem are available on the market: hormone replacement therapy, local vaginal treatments based on hormones (for example as disclosed in the document US 2005/209209) or substances of plant origin (soya isoflavones), and phytooestrogens (from hops, resveratrol, etc.), but none of the treatments acts solely at local level, because the action of said ingredients also takes place at systemic level, leading to the well-known side effects associated with hormonal action.

Some polymers are known to exert a protective action on the epithelia and the internal and external mucosa by creating a barrier to the action of external agents, such as irritants and pathogenic micro-organisms, in particular in parts of the body wherein the natural barrier, whether biochemical (such as mucus) or biological (such as bacterial microflora), is weakened.

The barrier function of polymers is created in two successive steps:
1) hydration of the polymers which absorb water and jellify, thereby acquiring the special plastic and adhesive characteristics necessary for coating and adhering to the target tissue to be protected,
2) mechanical dispersion of the hydrated polymer on the tissue to be protected,
by means of voluntary or involuntary movements of the area concerned.

Barrier polymers can be divided into two groups, depending on whether the hydration in water takes place spontaneously or is facilitated by a particular concentration of hydrogen ions (pH).

Carboxyvinyl polymers, Eudragit and chitosan belong to the second group, while natural gums, functionally modified celluloses (sodium carboxy-, methyl-, propyl-, hydroxypropyl-, hydroxyethyl, and ethylcellulose), polyvinylpyrrolidone and modified starches belong to the first group.

The polymer can be administered in pre-hydrated form in a liquid or as a solid which is hydrated locally at the site at which the barrier effect is to be exercised.

Said products are often used as barriers in pharmaceutical products for vaginal use, to protect against colonisation by pathogenic micro-organisms.

The most effective products can also contain Lactobacilli, already present in high concentrations in the vagina.

However, there is a very particular class of polymers that strongly attracts water without possessing a barrier action, namely polyethylene glycols.

Polyethylene glycols (PEGs, called macrogols in the pharmaceutical field) are a class of polymers widely used in a variety of pharmaceutical formulations, including formulations for parenteral or topical administration, ophthalmic, oral and rectal preparations.

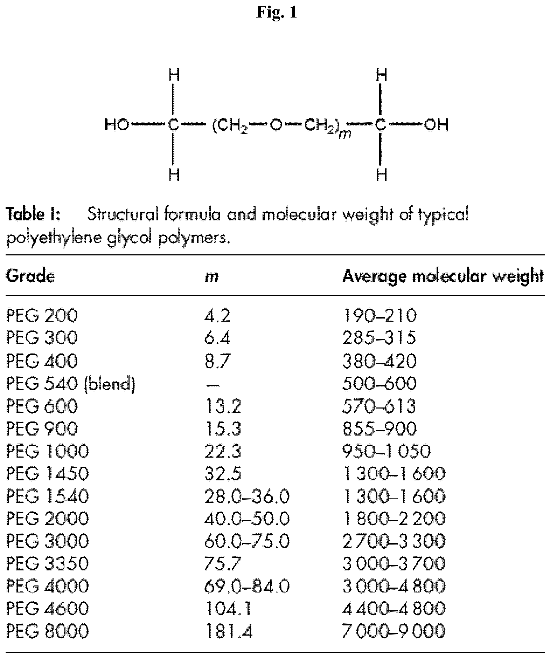

A formula of the monomer and the main grades of the polymer available for human use, classified by molecular weight, are illustrated in fig. 1.

Polyethylene glycols are stable hydrophilic substances which are essentially non-irritant to the skin.

The solid grades are used in creams and ointments, the consistency of which is adjusted by adding liquid grades of polyethylene glycol.

Mixtures of polyethylene glycols can be used as bases for suppositories, and possess many advantages over fats. For example, the melting point of the suppository can be higher so as to withstand exposure to warmer climates, and the suppositories are readily miscible with rectal fluids.

Aqueous solutions of polyethylene glycol can be used as suspending agents or to regulate the viscosity and consistency of other suspended carriers.

If used in combination with other emulsifiers, polyethylene glycols can act as emulsion stabilisers.

In solid formulations, the glycols with the highest molecular weights can improve the efficacy of the binders and confer plasticity on granules.

Polymers of grades 3350 and 4000 daltons are often used in the preparation of osmotic laxatives, which attract water and soften stools whose transit is delayed due to excessive concentration in the gastrointestinal tract, and have two interesting properties in the intestinal lumen:
1) they exert a considerable osmotic attraction on water,
2) they are not absorbed into the body through the intestine, and therefore act entirely in the intestinal lumen.

In fact, liquid polyethylene glycols can be absorbed when taken orally, but the polyethylene glycols with the highest molecular weight are not significantly absorbed by the gastrointestinal tract. The small amount of polyethylene glycol absorbed is mainly excreted unchanged in the urine.

### BRIEF DESCRIPTION OF FIGURES

Fig. 1. Main grades of macrogols for human use, classified by molecular weight.
Fig. 2 Some physical properties of macrogols, with particular details of the water content of PEG 4000. The viscosities for solid grades relate to 50% solutions in water.

### DESCRIPTION OF THE INVENTION

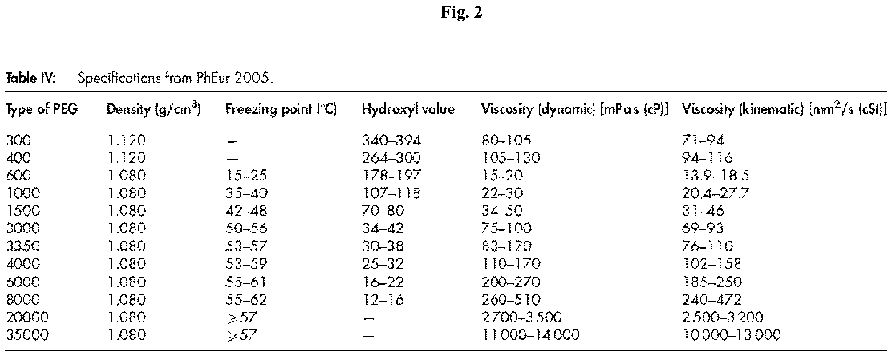

As shown in Fig. 2, PEGs are substantially highly hydrophilic. During some tests using PEGs of a grade higher than 3000 daltons, designed to restore the osmotic control of some galenical preparations intended for the vaginal mucosa (which typically contains small amounts of water), an interesting effect of said PEGs was surprisingly observed.

While it was expected that their use would cause water attraction useful for normal basal activities of the target site, where local administration was used, it has surprisingly been found that with the small amounts of water present in these areas after a week's administration, a stable improvement in the trophism of the mucosa took place, which lasted for several hours after administration.

This is probably not only due to the known attraction and retention of water effected by macrogols, but apparently due to the formation of a layer of secretions under normal conditions which are restored on contact with the mucosa, and which gives rise to a direct hydrating action on the tissues even at the end of the water attraction step.

Unlike the gastrointestinal use of macrogol as a laxative, there are no large amounts of water deriving from biological fluids in the vagina, only 1 or 2 ml at most, and there is no continuous progression of material to be digested, which could hinder the formation of a hypothetical hydration layer.

The observed effect on trophism takes place unexpectedly, depending on the physical properties; for example, if amounts of PEG ranging between 5 and 400 mg (or more particularly between 5 and 200 mg) are dissolved in 1-2 g of vaginal fluid (concentration 1-2%) with the viscosities shown in the table in fig. 2, high values are not observed for polymer grades 3350 and 4000 (80-170 mPa^{∗}s, for 50% solutions, which are slightly higher than those of water, amounting to about 50 mPa^{∗}s) or values compatible with a physical barrier layer.

The invention therefore relates to pharmaceutical compositions in solid form for the treatment or prevention of vaginal atrophy which contain, per dosage unit, between 5 and 400 mg of PEG of a grade exceeding 3000.

Higher doses may generate osmotic pressures which would almost certainly be too high for the mucosal cells, incompatible with their integrity and, as described in detail in the literature regarding osmotic concentrations, would induce cell lysis (osmotic pressure values exceeding 500 mOsm).

In one embodiment, the solid composition according to the invention contains between 5 and 200 mg of PEG per dosage unit.

In one embodiment, the solid composition according to the invention contains 25 mg of PEG per dosage unit.

In one embodiment, the solid composition according to the invention contains 200 mg of PEG with a grade exceeding 3000 per dosage unit.

In one embodiment, the solid composition according to the invention is a tablet or capsule.

In another embodiment, the PEG is selected from PEGs of grade 3350 and grade 4000.

To overcome the problem of partial or incomplete hydration of the polymer in the administration form and to accelerate the hydration rate, thus obtaining a faster benefit, surfactants can be added, i.e. substances that reduce the surface tension of the aqueous medium on the surfaces with which they come into contact, facilitating and standardising the wetting of said surfaces and therefore accelerating the penetration of the water required for hydration and dissolution of the polymer in the solid forms. Said substances reduce the surface tension of the viscous carrier, which is therefore diffused more readily at the interface. The presence of a surfactant at a low concentration therefore weakens the internal cohesive properties of the fluid, and at the same time strengthens the diffusion capacity of the polymer.

The safest and most commonly used surfactants include Span, Tween, polyoxyethylenated castor oil, sodium lauryl sulphate, the archetypal non-polymer anionic surfactant, and nonoxinols, which possess spermicidal properties.

A surfactant compound that is particularly well tolerated in the vaginal area is lauryl glucoside.

The vaginal compositions according to the invention can contain an acidifier useful to generate a correct pH for the area of administration. The preferred acidifier is lactic acid, which maintains an optimum pH in the vaginal fluid. Moreover, the acidity produced by the acid helps to inhibit the growth of various pathogenic microbes.

Lactic acid can be contained in quantities ranging from 15 to 50 mg per dosage unit.

To ensure rapid action, the solid compositions according to the invention can also contain disintegrating agents such as sodium carboxymethyl starch, which causes swelling on entry of the water in the dosage form, or polyvinylpolypyrrolidone, which generates deep channels, and/or a soluble diluent which helps to increase the penetration of the water into the solid form and hydration of the polymer.

Polyalcohols or sugars can be used as diluents, preferably in a readily soluble, often porous form, for example obtained by spray drying. The use of mannitol is preferred.

The mannitol can be contained in varying amounts of up to 900 mg per dosage unit.

The compositions according to the invention can also contain excipients that facilitate their production, selected from binders, lubricants and glidants.

The compositions according to the invention can contain other active agents useful for the homeostasis of the vaginal area, such as prebiotics, live or inactivated (tyndallised) probiotics.

The compositions can be prepared by methods known to the skilled person. A preparation process typically comprises the following steps:
- weighing of ingredients
- optional granulation in water or water and alcohol in a granulator/mixer followed by oven-drying of the granulate
- mixing with other excipients (diluent, glidants and lubricants).
- compression into tablets made with suitable tablet presses known to the art, or filling of hard gelatin capsules with a suitable capsule-filling machine.

The granulation step can be omitted if the materials used exhibit sufficient flowability and compressability characteristics in the capsule-filling machine.

### EXAMPLES

The examples below illustrate the invention in greater detail.

### EXAMPLE 1 - Composition according to the invention

Formulation: 1.1 g vaginal tablet
Daily dose: 1 tablet

| **Ingredient** | **mg / tab** | **FUNCTION** |
|---|---|---|
| Macrogol 3350 | 25.00 | Active ingredient |
| XYLO-oligosaccharides | 35.00 | prebiotic |
| Lactic acid | 50.50 | Acidifier |
| Lactobacillus plantarum (tyndallised) | 70.00 | Probiotic |
| Lauryl glucoside | 2.00 | Surfactant |
| Mannitol | 835.50 | Water-soluble diluent |
| Sodium carboxymethyl starch | 30.00 | Disintegrating agent |
| Polyvinylpolypyrrolidone | 30.00 | Disintegrating agent |
| Magnesium stearate | 20.00 | lubricant |
| Silicon dioxide | 2.00 | Glidant |

### Example 2 - Composition according to the invention

Formulation: 1.1 g vaginal tablet
Daily dose: 1 tablet

| **Ingredient** | **mg / tab** | **FUNCTION** |
|---|---|---|
| Macrogol 4000 | 200.00 | Active ingredient |
| Boric acid | 100.00 | Acidifier |
| Mannitol | 718.00 | Water-soluble diluent |
| Sodium carboxymethyl starch | 30.00 | Disintegrating agent |
| Polyvinylpolypyrrolidone | 30.00 | Disintegrating agent |
| Glyceryl behenate | 20.00 | lubricant |
| Lauryl glucoside | 2.00 | surfactant |

**Example 3 - Composition according to the invention**

Formulation: 0.35 g vaginal capsule, format "0"
Daily dose: 1 capsule

| **Ingredient** | **mg/capsule** | **FUNCTION** |
|---|---|---|
| Macrogol 3350 | 25.00 | Active ingredient |
| XYLO-oligosaccharides | 35.00 | prebiotic |
| Lactic acid | 50.00 | Acidifier |
| Lactobacillus plantarum (tyndallised) | 70.00 | Probiotic |
| Lauryl glucoside | 2.00 | Surfactant |
| Gelatin | 90.00 | Outer shell |
| Sodium carboxymethyl starch | 30.00 | Disintegrating agent |
| Polyvinylpolypyrrolidone | 30.00 | Disintegrating agent |
| Magnesium stearate | 20.00 | lubricant |
| Silicon dioxide | 2.00 | surfactant |

### Example 4 - Effect on trophism

As shown in the table 1, which reports the VIH scale (Vaginal Health Index, G. Bachmann. Urogenital ageing: an old problem newly recognized. Maturitas 22 Supp. 1995, S1-85*,* wherein the lower the score, the greater the atrophy), the level of vaginal secretions is considered to be a good index of vaginal functionality, which is closely connected with the trophism of the vaginal mucosa.

Vaginal Health Index *(*G. Bachmann. Urogenital ageing: an old problem newly recognized. Maturitas 22 Supp. 1995, S1-85*)*

**TABLE 1**

| **Score** | **Elasticity** | **Secretions** | **pH** | **Appearance of mucosa** | **Humidity** |
|---|---|---|---|---|---|
| 1 | None | None | >6 | Petechiae | None, inflamed mucosa |
| 2 | Poor | Slight, yellow | 5.6-6.0 | Bleeds slightly on contact | None, mucosa not inflamed |
| 3 | Sufficient | Superficial, white | 5.1-5.5 | Bleeds when rubbed | Minimal |
| 4 | Good | Moderate, white | 4.7-5.0 | Thin, non-friable mucosa | Moderate |
| 5 | Excellent | Normal | ≤4.6 | Normal mucosa | Normal |

Moreover, the pH of the fluid in the vaginal environment indicates the correct functionality of the tissue when its value is slightly acid.

The composition described in example 1 was administered daily for 10 days to 6 menopausal women who complained of vaginal atrophy, and the amount of fluids present in the vagina was verified in the daytime, well after administration, which took place the previous evening, by measuring the weight increase of an absorbent swab for microbiological tests after being introduced into the vagina and left there for 1 minute.

A pH litmus test strip with a specific graduation for urine was applied to the same swab (pH values detectable: 4.5; 5.0; 5.5; 6.0; 6.25; and 6.5).

The results, set out in the table 2, demonstrate that the treatment significantly improves the parameters that denote good trophism of the mucosa, i.e. quantity of secretion and the corresponding pH.

**TABLE 2**

| **Subject No.** | **Age** | **Secretion absorbed** | | | | | |
|---|---|---|---|---|---|---|---|
| | | **Start of treatment** | | **after 5 days** | | **after 10 days** | |
| | | **(mg)** | **pH** | **(mg)** | **pH** | **(mg)** | **pH** |
| 1 | 49 | 0.1 | 6 | 0.2 | 5.5 | 0.5 | 5 |
| 2 | 39 | 0.1 | 5.75 | 0.3 | 5.5 | 0.4 | 5.5 |
| 3 | 54 | 0.0 | nd | 0.1 | 6 | 0.2 | 5.7 |
| 4 | 51 | 0.3 | 5.5 | 0.5 | 5.5 | 0.6 | 5.0 |
| 5 | 47 | 0.2 | 5.5 | 0.7 | 4.5 | 0.7 | 4.5 |
| 6 | 56 | 0.1 | 6 | 0.3 | 5.5 | 0.4 | 5.5 |

| | | | | | | | |
|---|---|---|---|---|---|---|---|
| N/D not measurable | | | | | | | |

## Claims

1. Pharmaceutical composition in solid form comprising, per dosage unit, between 5 and 400 mg of a PEG with a grade of 3000 or more, for use in the topical treatment or prevention of vaginal atrophy.

2. Composition for use according to claim 1, wherein the PEG is selected from PEG 3350 and PEG 4000.

3. Composition for use according to claim 1 or 2, wherein the solid form is selected from tablets and capsules.

4. Composition for use according to claims 1 to 3, further comprising an ingredient selected from a surfactant, an acidifying agent, a disintegrating agent and a water-soluble diluent.

5. Composition for use according to claim 4, wherein the surfactant is lauryl glucoside.

6. Composition for use according to claim 4, wherein the acidifying agent is lactic acid.

7. Composition for use according to claim 4, wherein the disintegrating agent is a mixture of sodium carboxymethyl starch and polyvinylpolypyrrolidone.

8. Composition for use according to claim 4, wherein the water-soluble diluent is mannitol.

## Patentansprüche

1. Pharmazeutische Zusammensetzung in fester Form, umfassend pro Dosierungseinheit zwischen 5 und 400 mg eines PEG mit einem Grad von 3000 oder mehr zur Verwendung bei der topischen Behandlung oder Prävention von Vaginalatrophie.

2. Zusammensetzung zur Verwendung nach Anspruch 1, wobei das PEG ausgewählt ist aus PEG 3350 und PEG 4000.

3. Zusammensetzung zur Verwendung nach Anspruch 1 oder 2, wobei die feste Form ausgewählt ist aus Tabletten und Kapseln.

4. Zusammensetzung zur Verwendung nach den Ansprüchen 1 bis 3, ferner umfassend einen Inhaltsstoff, ausgewählt aus einem Tensid, einem Säuerungsmittel, einem Zerfallsmittel und einem wasserlöslichen Verdünnungsmittel.

5. Zusammensetzung zur Verwendung nach Anspruch 4, wobei das Tensid Laurylglucosid ist.

6. Zusammensetzung zur Verwendung nach Anspruch 4, wobei das Säuerungsmittel Milchsäure ist.

7. Zusammensetzung zur Verwendung nach Anspruch 4, wobei das Zerfallsmittel eine Mischung aus Natriumcarboxymethylstärke und Polyvinylpolypyrrolidon ist.

8. Zusammensetzung zur Verwendung nach Anspruch 4, wobei das wasserlösliche Verdünnungsmittel Mannit ist.

## Revendications

1. Composition pharmaceutique sous forme solide comprenant, par unité posologique, entre 5 et 400 mg d'un PEG avec un grade de 3000 ou plus, pour une utilisation dans le traitement topique ou la prévention de l'atrophie vaginale.

2. Composition pour une utilisation selon la revendication 1, dans laquelle le PEG est choisi parmi le PEG 3350 et le PEG 4000.

3. Composition pour une utilisation selon la revendication 1 ou 2, dans laquelle la forme solide est choisie parmi des comprimés et des capsules.

4. Composition pour une utilisation selon les revendications 1 à 3, comprenant en outre un ingrédient choisi parmi un tensioactif, un agent acidifiant, un agent de désintégration et un diluant hydrosoluble.

5. Composition pour une utilisation selon la revendication 4, dans laquelle le tensioactif est le lauryl glucoside.

6. Composition pour une utilisation selon la revendication 4, dans laquelle l'agent acidifiant est l'acide lactique.

7. Composition pour une utilisation selon la revendication 4, dans laquelle l'agent de désintégration est un mélange de carboxyméthyl amidon sodique et de polyvinylpolypyrrolidone.

8. Composition pour une utilisation selon la revendication 4, dans laquelle le diluant hydrosoluble est le mannitol.
